Europäisches Patentamt

European Patent Office

Office européen des brevets

(11) Publication number: **0 560 275 A1**

## EUROPEAN PATENT APPLICATION

(21) Application number: **93103740.2**

(22) Date of filing: **09.03.93**

(51) Int. Cl.5: **A61K 31/205**

(30) Priority: **11.03.92 IT RM920165**

(43) Date of publication of application:
**15.09.93 Bulletin 93/37**

(84) Designated Contracting States:
**AT BE CH DE DK ES FR GB GR IE IT LI LU MC NL PT SE**

(71) Applicant: **Sigma-Tau Industrie Farmaceutiche Riunite S.p.A.**
**Viale Shakespeare, 47**
**I-00144 Roma(IT)**

(72) Inventor: **de Simone, Claudio, Dr.**
**Via Nuoro, 14, Lotto 1069 Nuova Florida**
**I-00040 Ardea - Roma(IT)**

(74) Representative: **La Ciura, Salvatore**
**Via Francesco Sforza, 3**
**I-20122 Milan (IT)**

(54) Use of L-carnitine for treating carnitine-depleted HIV-seropositive patients.

(57) A novel therapeutic utilization of L-carnitine and the pharmacologically acceptable salts thereof for treating carnitine-depleted HIV-seropositive patients, is disclosed.

EP 0 560 275 A1

The present invention relates to a novel therapeutic utilization of L-carnitine and its pharmacologically acceptable salts for the therapeutic treatment of carnitine-depleted HIV-seropositive patients.

Previous therapeutical uses of L-carnitine are already known.

For instance, L- carnitine has been used in the cardiovascular field in the treatment of acute and chronic myocardial ischaemia, angina pectoris, cardiac arrhythmias and insufficiency. In nephrology, L-carnitine has been administered to chronic uraemic patients who are subject to regular haemodialysis treatment with a view to counteracting muscular asthenia and the onset of muscular cramps.

Further therapeutical uses are the restoration of HDL/LDL + VLDL ratio to normal and in total parenteral nutrition.

However, there is no relationship between the foregoing known therapeutical utilizations of L-carnitine and the utilization which is the object of the present invention.

In AIDS subjects cardiac symptoms and muscle weakness have been attributed to drug toxicity, altered immunologic mechanisms and nutritional deficiency in addition to HIV infection. Many of these patients are also hypometabolic as compared with healthy subjects.

In these patients wasting sindrome and cachexia are common manifestations. Since like symptoms - skeletal myopathy or cardiomiopathy or both - have been described in carnitine-depleted patients, also HIV-seropositive patients would have been expected to similarly exhibit carnitine deficiency. As known, L-carnitine is necessary for the translocation of fatty acids into the mitochondria where beta-oxidation takes place.

However, in a study by J.D.Bogden et al "Micronutrient status and Human immunodeficiency Virus (HIV) infection", published in Annals New York Academy of Sciences, 1990, 537, 189-195, entirely different results from those that could have been expected, were reported. The primary objective of Bogden et al study was to determine the prevalence of abnormalities of plasma micronutrient concentrations in HIV-seropositive subjects. A further objective was to compare plasma micronutrient concentrations in asymptomatic HIV-seropositive subjects with those of subjects with acquired immune deficiency syndrome (AIDS) and with AIDS-related complex (ARC). As regards carnitine, Bogden et al study reached opposite conclusions to those expected: not only no one of the enrolled patients exhibited carnitine levels below normal values, but a remarkable percentage thereof (37%) showed carnitine levels higher than normal (see particularly table 2, page 191 of the aforesaid article).

Also because the authors of this study are authoritative researchers, it is apparent that the study results could have not but deterred the average skilled expert from conducting researches on a prospective therapeutic role of L-carnitine for treating HIV-seropositve patients.

Surprisingly, it has now been found (although the misleading conclusions which might be induced by the aforesaid article) that not only most of the HIV-seropositive subjects show L-carnitine levels markedly below the normal value, but that surprisingly effective therapeutic results, which will be hereinbelow reported, are achieved in these subjects by L-carnitine administration.

A clinical study was conducted, 29 AIDS patients aged 27 to 41 years, mean ± s.d. 32±5 years, were enrolled in the study. These patients, who had a previous history of drug addiction by intravenous self-administration, were administered zidovudine (AZT), 500-800 mg/day for 8±6 months; (range 2-28 months). No patient presented abnormal serum creatine kinase levels or signs and symptoms of muscle or cardiac dysfunction (i.e., no enlargement or peaking of the T waves, increased ventricular wall thickness or hypertrophy on echocardiogram analysis). Their nutritional status was rated as satisfactory or fair. The control group consisted of 14 healthy individuals, matched for age and sex.

L-carnitine was evaluated by the radioenzyme method described by J. Cederblad and S. Lindsted in Clin. Chim. Acta 37, 235-243 (1972).

Briefly, a neutralized perchloric acid extract of plasma was incubated with $^{14}$C-acetyl-CoA in the presence of carnitine acetyltransferase (CAT). The labelled acetyl-L-carnitine thus formed was separated from the unreacted $^{14}$C-acetyl-CoA by an anion exchange resin, and radioactivity counts were evaluated in the supernatant. The mean ± s.d. of total L-carnitine was 43±9 $\mu$ M/L (maximum value 74, minimum value 20 $\mu$ M/L) versus 53±6 $\mu$ M/L (maximum value 61, minimum value 47) in the control group.

Levels of total L-carnitine below the values of the control group were observed in 21 (72%) AIDS patients, whereas only four patients (14%) showed higher levels of total L-carnitine compared to normal controls.

These results are clearly in disagreement with those of the aforesaid study.

Furthermore, the L-carnitine effect on DNA cycle in HIV-infected lymphocytes was assessed. The tissue culture medium was RPMI 1640 supplemented with L-glutamine (200 mM), 5% foetal calf serum, penicillin (200 IU/ml) and streptomycin (100 $\mu$g/ml). L-carnitine (Carnitene®, Sigma-Tau, Pomezia, Italy) was added from the start of the culture (O h) at concentrations of 100 and 200 $\mu$g/ml. Mononuclear cells were separated from heparinized peripheral blood of 21 AIDS patients (mean age 35±7 years) by density gradient centrifugation over Ficoll-Hypaque. The cells were washed three times in tissue culture medium, prior to culture in sterile round bottom tubes. Each tube contained 1 ml cell suspension ($2 \times 10^6$ cells/ml), 25 $\mu$l L-carnitine and 25 $\mu$l phytohemagglutinin (PHA) at an optimal stimulating concentration of 5 $\mu$g/ml. The tubes were incubated at 37°C in 5% $CO_2$ for 2 days.

Our results show that 10 patients (47,6%) were hyporesponsive to PHA stimulation (> 20% cells in S and $G_2$-M phases) and the remaining 11 (52,4%) were normoresponsive. L-carnitine (100 and 200 $\mu$g/ml) ameliorated (p>0,01) the PHA-driven lymphocyte proliferative responses in hyporesponsive subjects.

TABLE I. Summary of the percentage of cells in S and $G_2$-M phases in 10 hyporesponsive and 11 normoresponsive AIDS patients. Peripheral blood mononuclear cells were stimulated with phytohemagglutinin (PHA; 5 $\mu$g/ml) or with PHA + L-carnitine (100 and 200 $\mu$g/ml) for 48 hours.

| % in S and $G_2$-M* phases | PHA | PHA + Carnitine 100 $\mu$g/ml | PHA + Carnitine 200 $\mu$g/ml |
|---|---|---|---|
| Hyporesponsive** (n° 10) | 9.2±3.1 | 12.5±3.5° | 12.4±3.0° |
| Normoresponsive (n° 11) | 32.5±9.3 | 30.6±6.7 | 30.8±7.4 |

\* Data are expressed as mean percentage ± SD

\*\* < 20% cells in S and $G_2$-M phases following PHA stimulation °p<0.01.

Whereas for the aforesaid, already known therapeutical utilization, 10 to 30 mg L-carnitine/kg body weight/day) is the suitable dose to be administered, it was found that, having regard to the novel utilization which is the object of this invention, the dose to be administered is at least 80 mg. preferably 100-140 mg. L-carnitine/kg body weight/day or an equivalent amount of a pharmacologically acceptable salt thereof. Since L-carnitine is practically non-toxic, if need be, even larger doses can be safely administered.

L-carnitine is formulated with the usual excipients used for preparing orally or parenterally administrable compositions.

Pharmaceutical compositions in unit dosage form suitable for the aforesaid therapeutical utilization comprise from about 1000 to about 2000 mg L-carnitine or an equivalent amount of a pharmacologically acceptable salt thereof.

Non-limiting examples of pharmacologically acceptable salts are: cloride; bromide; iodide; aspartate, particularly acid aspartate; citrate, particularly acid citrate; tartrate; phosphate, particularly acid phosphate; fumarate, particularly acid fumarate; glicerophosphate; glucosephosphate; lactate; maleate, particularly acid maleate; orotate; oxalate, particularly acid oxalate; sulphate, particularly acid sulphate; trichloroacetate; trifluoroacetate and metansulphonate.

**Claims**

1. Use of L-carnitine or a pharmacologically acceptable salt thereof for producing a medicament for the therapeutical treatment of HIV-seropositive subjects.

2. An orally or parenterally administrable pharmaceutical composition, in unit dosage form, for treating HIV-seropositive subjects, comprising from 1000 to 2000 mg L-carnitine or an equivalent amount of a pharmacologically acceptable salt thereof, and a pharmacologically acceptable eccipient therefor.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | IMMUNOPHARMACOLOGY AND IMMUNOTOXICOLOGY vol. 15, no. 1, January 1993, pages 1 - 12 DE SIMONE C. ET AL 'HIGH DOSE L-CARNITINE IMPROVES IMMUNOLOGIC AND METABOLIC PARAMETERS IN AIDS PATIENTS' * the whole document * | 1,2 | A61K31/205 |
| X | AIDS vol. 6, no. 2, February 1992, pages 203 - 205 DE SIMONE, C. ET AL 'L-CARNITINE DEFICIENCY IN AIDS PATIENTS' * the whole document * | 1,2 | |
| A | US-A-4 415 588 (CAVAZZA) 15 November 1983 * the whole document * * especially column 4, line 1-column 5, line 16 * | 1,2 | |
| X | GB-A-2 052 976 (DR EDUARD FRESENIUS CHEMISCHPHARMAZEUTISCHE INDUSTRIE KG) 4 February 1981 * the whole document * * especially page 3,line 31-33 * | 2 | **TECHNICAL FIELDS SEARCHED (Int. Cl.5 )** A61K |
| A | CLINICAL NUTRITION vol. 6, no. 1, February 1987, pages 39 - 44 CARLSSON, M. ET AL 'L-CARNITINE ENHANCES LYMPHOCYTE MITOGENESIS IN DEPLETED TRAUMATISED AND INFECTED PATIENTS' * the whole document * | 1,2 | |

The present search report has been drawn up for all claims

| Place of search | Date of completion of the search | Examiner |
|---|---|---|
| THE HAGUE | 17 JUNE 1993 | MAIR J. |

EPO FORM 1503 03.82 (P0401)